# EUROPEAN PATENT APPLICATION

(11) **EP 3 315 953 A1**
(43) Date of publication of application: **02.05.2018**
(21) Application number: 16382504.5
(22) Date of filing: 31.10.2016
(51) Int. Cl.: G01N 21/95

(54) **OPTICAL INSPECTION SYSTEM OF OBJECTS DESTINED TO BE USED IN A QUALITY CONTROL SYSTEM IN A SERIES MANUFACTURING PROCESS AND ASSOCIATED METHOD**

(71) Applicant: Twoptics Systems Design SL, 08034 Barcelona (ES)
(72) Inventor: SIEGEL, Thomas, 08034 Barcelona (ES); CIFUENTES FERNÁNDEZ, Andrés Fernando, 08034 Barcelona (ES)
(74) Representative: Balder IP Law, S.L.

(57) **Abstract**

Optical inspection system of objects (O) destined to be used in a control quality system in a series manufacturing process, which comprises an optical coherence tomography device (1) provided with a low coherence source (11), an objective lens (12) so that a focal plane (13) is defined where the part of the object (O) to be inspected has to be placed, an imaging device for acquiring images of the object (0), the imaging device (2) being provided with a camera (21), the imaging device comprising a beam splitter (22), the beam splitter (22) being arranged between the objective lens (12) and the focal plane (13), the beam splitter (22) being arranged to reflect part of the light coming from the object (O) and direct it toward the camera (21), wherein the device optical coherence tomography (1) comprises a lens system provided with one or more mobile reflectors (14) and intended to allow sweeps with the laser beam (11) on the object (O), and wherein the system comprises a platform (3) for supporting the object (O) and means for moving the platform (3) with respect to the optical coherence tomography (1) device, so that it is possible to perform an inspection of areas identified by the camera (21) on the object (O) by alternating displacement of the beam by the reflector (14) and by moving the platform (3).

## Description

### TECHNICAL FIELD

The present invention is concerned with the high-speed inspection of manufactured objects with three dimensional feature sizes ranging from one micron to a few millimetres in depth. The gain in efficiency comes from the splitting of the data acquisition in two sets of data through two separate measurement devices, one acquiring two-dimensional planar data and the other acquiring the depth, the third dimension of the object under inspection.

This invention allows the measurement of surface three dimensional features in all materials and full depth profiling in transparent and turbid materials, allowing the characterisation of embedded features.

### STATE OF THE ART

Known are in the art the optical inspection system of objects destined to be used in a control quality system in a series manufacturing process, which comprises an optical coherence tomography device provided in turn with:
- a low coherence source;
- a beam splitter sending part of the beam from the low coherence source to a reference arm and the other part to the manufactured object;
- an objective lens so that a focal plane of the low coherence source is defined where the part of the object to be inspected has to be placed;
- an imaging device for acquiring images of the object, the imaging device being provided with a camera;
- the imaging device comprising a beam splitter;
- the beam splitter being arranged between the objective lens and the focal plane;
- the beam splitter being arranged to reflect part of the light coming from the object and direct it toward the camera;
- wherein the device optical coherence tomography comprises one or more mobile reflectors and an objective intended to allow sweeps with the laser beam on the object; - a spectrometer to which the recombined beams from the manufactured object and the reference arm are sent and which records their interference from which is extracted the depth profile along the low coherence beam.

A device having these components is described for example in the European patent application EP 95114600 from Carl Zeiss, relative to a "coherence tomography corneal mapping optical apparatus".

As its title indicates, this is a device specially designed for application in the ophthalmological sector.

In this sector a maximum tomography definition has to be ensured, because any deviation can have serious consequences for the patient.

This implies long exploration times and intensive use of memory to store the data associated with the images. For example, an object having a size of 100 cm² can involve half an hour of inspection for generating a 300 Gb data archive, considering a powerful processor as those currently available.

Moreover, artificial vision devices are known for quality control 100% of the parts in manufacturing processes that involve imaging of each of the parts to be verified and compare them with a reference pattern. In these systems an optimal compromise between accuracy of verification and the associated cost have to be achieved.

Therefore, the object of the present invention is to resolve a current bottleneck in inspection rate of three dimensional micro-featured objects. Current inspection methods of three dimensional micro-featured goods including, but not limited to, micro-fluidic chips are slow and/or destructive, and are unable to match the current production rates of the manufacturers.

### DESCRIPTION OF THE INVENTION

For overcoming the prior art drawbacks, the present invention proposes an optical inspection system of objects destined to be used in a control quality system in a series manufacturing process, which comprises an optical coherence tomography device provided in turn with:
- a low coherence source;
- an objective lens so that a focal plane is defined where the part of the object to be inspected has to be placed;
- an imaging device for acquiring images of the object, the imaging device being provided with a camera;
- the imaging device comprising a beam splitter;
- the beam splitter being arranged between the objective lens and the focal plane;
- the beam splitter being arranged to reflect part of the light coming from the object and direct it toward the camera;
- wherein the device optical coherence tomography comprises a lens system provided with one or more mobile reflectors and intended to allow sweeps with the laser beam on the object;
wherein the system comprises a platform for supporting the object and means for moving the platform with respect to the optical coherence tomography device, so that it is possible to perform an inspection of areas identified by the camera on the object by alternating displacement of the beam by the reflector and by moving the platform.

These features allow carrying out an efficient inspection of all the micro-featured goods produced in a manufacturing process. This highly efficient inspection rate is achieved through the combination of in-plane diagnostic through the use of machine vision in two dimensions, possible thanks to the camera, with depth measurement, third dimension, through the use of broadband low coherence interferometry and with the combination of sweeps through two differentiated mechanisms, that is, the combination of the mobile reflector and lens system itself, and a movable platform.

The separation of the characterisation of the different dimensions using separate measurement devices allows a significant reduction of the data load as compared with full volumetric imaging, hence reducing the inspection time and making possible the inspection of 100% of the products.

Moreover, microfluidic items are widely used in the medical sector, for example for detecting ion species in biological fluids, and the present invention allows inspecting all the manufactured items without contact, which allows to prevent contamination.

In some embodiments, the lens system comprises two motors for allowing sweeps in two dimensions.

The focal distance of the lens system is higher than 25 mm. This feature allows to place the beam splitter after the full scanning system which is made up of the mobile reflector and lens system.

The inventors have found that this large focal distance implies that the OCT system has too poor a lateral resolution to be able to quantify accurately the two planar dimensions of the micro-features of the manufactured object. However, the measurement of the planar dimensions is here performed by the machine vision, unlike in traditional OCT systems. The focal spot of the OCT remains sufficiently small to enter even high aspect ratio features present in the object.

In some embodiments the source is a laser.

In some embodiments the platform is movable in three directions.

In some embodiments the system comprises a window provided in its edge with an illumination system destined to light the sample for the camera and a transparent cover to prevent dust entering the measurement head.

The invention also relates to a method for optical inspection system in a control quality system in a series manufacturing process, wherein the optical inspection system according to any of the previous claims is used, comprising the steps of:
a) Place an object to be inspected in the focal plane;
b) Acquire an image of the object;
c) Identify the area to be scanned of the object;
d) Partition the areas to be scanned in subareas, such that the subareas can be swept by the laser by the only use of the reflector using the image of the object;
e) Place a subarea in the field of view of the optical coherence tomography device using the platform;
f) Scan the subarea by sweeping with the reflector to measure depth profiles of preselected features.
g) Use the camera to perform the lateral measurements of the features in the sub-area;
h) Displace the object with respect to the optical coherence tomography-machine vision device by moving the platform to place another subarea of the object in the field of view of the optical coherence tomography - machine vision device;
i) Iterate steps f) to h) until all the subareas making the identified area have been scanned.

Preferably, the image of the object is a CAD drawing.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 is a schematic diagram of the device main components of the optical coherence tomography device according to the invention.
Figure 2 is a schematic representation of an OCT beam inspecting a microfluidic channel.

### DESCRIPTION OF A WAY OF CARRYING OUT THE INVENTION

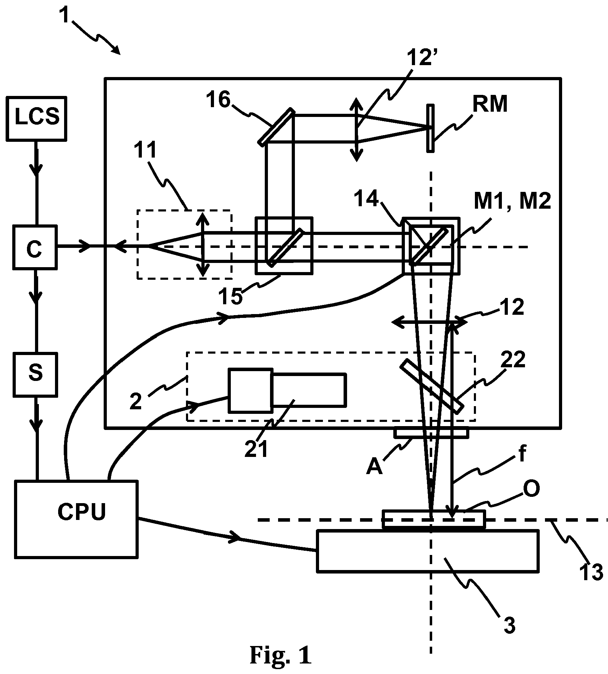

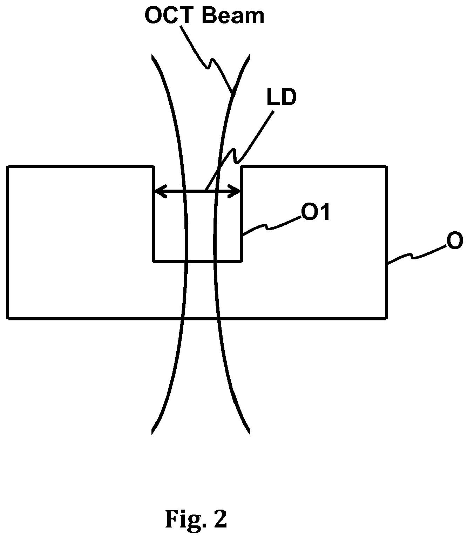

As shown in FIG. 1, the present invention relates to an optical inspection system of objects O destined to be used in a control quality system in a series manufacturing process, for example a microfluidic device provided with micro-channels 01 as shown in FIG. 2.

As shown in FIG. 1, the object or objects O to be scanned must be placed under the window A.

The optical coherence tomography device 1 of the system is provided with:
- a low coherence source LCS, which can be located in the same housing itself, for example directly coupled to the port 11, or, as shown, being remotely located, and driven by means of optical fibers to a port 11;
- an objective lens 12 so that a focal plane 13 is defined. The focal distance is f as shown in FIG. 1.
- the device also includes a circulator C and a spectrometer S.

The part of the object O to be inspected has to be placed such that it coincides with the focal plane 13.

The system 1 is provided with an imaging device 2 for acquiring images of the object O, the imaging device 2 being provided with a camera 21. This camera is part of a machine vision system able to identify the features 01 of the object O to be scanned.

The imaging device comprises a beam splitter 22 arranged between the objective lens 12 and the focal plane 13 and is arranged to reflect part of the light coming from the object O and direct it toward the camera 21.

Therefore, there is no camera present which can see the full object, but instead the camera for machine vision shares the same field of view as the OCT.

A focusing beam going through a piece of glass induces spherical aberration, which net result is a loss of lateral resolution, a larger spot if talking in terms of a laser. In many systems used for ophthalmologic systems the OCT beam is used in reflection since this sets the lateral resolution of the system and the reflection does not introduce aberrations.

The camera is typically used in transmission since it is merely used to give a rough image of the sample O. In the present case the camera 21 is doing the lateral dimensioning. An aim of the present invention is to reduce any aberration on that path, such that the camera 21 is used in reflection.

The spot of the OCT beam used in transmission according to the present invention and as shown in FIG. 2 is already big since the longer focal length is used, so that the small amount of spherical aberration affects only slightly.

The optical coherence tomography 1 comprises a scanning system provided with one or more mobile reflectors, which may or may not be actuated by galvonometric motors M1, M2, and a set of lenses 12. The scanning system allows the low coherence beam 11 to be scanned on the object O. 12' is an objective lens, identical to the objective lens 12, destined to ensure that both arms have the same length and amount of material for dispersion matching. 15 is the beam splitter of the interferometer and 16 is a mirror just provided for compactness purposes.

As shown schematically in the lower part of FIG. 1, the system comprises a platform 3 for supporting the object O and means for moving the platform 3 with respect to the optical coherence tomography 1 device.

Therefore, it is possible to perform an inspection of areas identified by the camera 21 on the object O by alternating displacement of the beam by the reflector 14 and by moving the platform 3, which can be moved following up to three dimensions.

The housing comprises in a wall a window A provided in its edge with an illumination means, for example a LED ring, destined to light the sample for the camera 21 and a transparent cover to prevent dust entering the measurement head.

This system allows carrying out a method for optical inspection system in a control quality system in a series manufacturing process, comprising the steps of:
a) Place an object O to be inspected in the focal plane 13;
b) Acquire an image of the object O with the camera 21;
c) Identify the area to be scanned of the object O;
d) Partition the areas to be scanned in subareas, such that the subareas can be swept by the laser by the only use of the reflector 14;
e) Place a subarea in the field of view of the optical coherence tomography device 1 using the platform 3;
f) Scan the subarea by sweeping with the reflector 14 to measure depth profiles in selected regions
g) Use the camera 21 to perform the lateral measurements of the object O;
h) Displace the object O with respect to the optical coherence tomography device 1 by moving the platform 3 to place another subarea of the object O in the field of view of the optical coherence tomography device 1;
i) Iterate steps f to h until all the subareas making the identified area have been scanned.

For example, M1 and M2 allow a fast sweep over features within the field of view of the instrument whereas the platform allows a sweep of the object from one subarea of the size of the field of view to the next.

Therefore, the present invention is directed to the combination of two metrology techniques, SD-OCT and machine vision for improved inspection rates.

SD-OCT is an interferometric technique whereby a beam of light is split in two using a beam-splitter 15. One half of the light is directed towards a telecentric scanning system which may scan the focussed beam onto the 3D object O under inspection hereafter referred to as the sample O. The other half of the beam is sent to a reference mirror RM along a path equivalent to that going to the sample O. The light reflected from the reference mirror RM and that scattered from the sample O are recombined by the same beam-splitter 15.

The combined beam is directed to a spectrometer S where the interference between the two beams is recorded using a linear pixelated detector. This recorded interference pattern is Fourier transformed in the CPU to yield the depth profile of the sample O. The retrieved dimensions may be compared to dimensional specifications for pass/fail testing of the sample O.

The machine vision is an imaging technique whereby the sample is imaged preferably onto a 2D pixel array detector 21. The image is used by software to recognise features. Once recognised, the dimensions of features on the sample O may be measured by the computer CPU through the machine vision. The retrieved dimensions may be compared to dimensional specifications for pass/fail testing of the sample O.

The galvonometric scanning system M1, M2, 14 is much faster but with a much reduced field of view than the XY stage allowed by the platform 3, it therefore allows fast scanning of the features on the sub-area being inspected.

To achieve the above object, the main aspect of this invention is the separation of the measurement of the 3 dimensions between two different metrology technologies: machine vision to do the lateral dimensioning and OCT to measure the depth profiles.

To achieve the above object, the OCT and the MV share the same field of view. The combination of the fields of view may be done using a dichroic beam splitter 22 and using different wavelength bands for the OCT and for the MV.

The combination of the fields of view may also be done using a standard beam splitter. The OCT beam may be scanned across the field of view using, but not limited to, a telecentric system with two galvonometric motors M1, M2 to drive the motion of the light beam.

To achieve the above object, the machine vision plays a dual role. Its first role is to perform the lateral dimensioning of all features within the field of view from a single snapshot (two dimensions are covered by the machine vision).

Its second role is to locate the features where the depth information is critical and send to the OCT scanning system the coordinates within the field of view of the features to be inspected in the third dimension (defined as depth).

To achieve the above object, the OCT beam measures the depths profiles of user-defined features.

To achieve the above object, the lateral dimensioning measured by the machine vision and the depth profile of the user-defined features are compared to the design intent and the design tolerance of the sample manufacturer.

The galvonometric motors M1, M2 scan the beam to the position of the feature on the sample plane 13, the beam allows the extraction of the depth at the point where it is incident on in the plane. No lateral information leading to a pass/fail test is extracted from that measurement, the lateral information is extracted from the image from the machine vision 21 only.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the invention is obviously not limited to the specific embodiment described herein, but also encompasses any variations that may be considered by any person skilled in the art (for example, as regards the choice of materials, dimensions, components, configuration, etc.), within the general scope of the invention as defined in the claims.

## Claims

1. Optical inspection system of objects (O) destined to be used in a control quality system in a series manufacturing process, which comprises an optical coherence tomography device (1) provided in turn with:
- a low coherence source (11);
- an objective lens (12) so that a focal plane (13) is defined where the part of the object (O) to be inspected has to be placed;
- an imaging device for acquiring images of the object (O), the imaging device (2) being provided with a camera (21);
- the imaging device comprising a beam splitter (22);
- the beam splitter (22) being arranged between the objective lens (12) and the focal plane (13);
- the beam splitter (22) being arranged to reflect part of the light coming from the object (O) and direct it toward the camera (21);
- wherein the device optical coherence tomography (1) comprises a lens system provided with one or more mobile reflectors (14) and intended to allow sweeps with the laser beam (11) on the object (O);
**characterised in that** the system comprises a platform (3) for supporting the object (O) and means for moving the platform (3) with respect to the optical coherence tomography (1) device, so that it is possible to perform an inspection of areas identified with the camera (21) on the object (O) by alternating displacement of the beam by the reflector (14) and by moving the platform (3).

2. Optical inspection system according to claim 1, wherein the lens system comprises two motors (M1, M2) for allowing sweeps in two dimensions.

3. Optical inspection system according to claim 2, wherein the focal distance (f) of the lens system is higher than 25 mm.

4. Optical inspection system according to any of the previous claims, wherein the source is a laser.

5. Optical inspection system according to any of the previous claims, wherein the platform (3) is movable in three directions.

6. Optical inspection system according to any of the previous claims, which comprises a window (A) provided in its edge with an illumination system destined to light the sample for the camera (21) and a transparent cover.

7. Method for optical inspection system in a control quality system in a series manufacturing process, wherein the optical inspection system according to any of the previous claims is used, comprising the steps of:
a) Place an object (O) to be inspected in the focal plane (13);
b) Acquire an image of the object (O);
c) Identify the area to be scanned of the object (O);
d) Partition the areas to be scanned in subareas, such that the subareas can be swept by the laser by the only use of the reflector (14);
e) Place a subarea in the field of view of the optical coherence tomography device (1) using the platform (3);
f) Scan the subarea by sweeping with the reflector (14) to measure depth profiles in selected regions
g) Use the camera (21) to perform the lateral measurements of the object (O);
h) Displace the object (O) with respect to the optical coherence tomography device (1) by moving the platform (3) to place another subarea of the object (O) in the field of view of the optical coherence tomography device (1);
i) Iterate steps f) to h) until all the subareas making the identified area have been scanned.
